# EUROPEAN PATENT APPLICATION

(11) **EP 1 574 575 A1**
(43) Date of publication of application: **14.09.2005**
(21) Application number: 03813300.5
(22) Date of filing: 02.12.2003
(51) Int. Cl.: C12N 15/12, C12N 5/10, C12Q 1/68, C07K 14/705, C07K 16/28, C12P 21/02, C12Q 1/02, G01N 33/15, G01N 33/50, G01N 33/53, G01N 33/566, G01N 37/00, A01K 67/027, A61K 31/7088, A61K 38/00, A61K 48/00, A61P 3/10, A61P 13/12, A61P 43/00

(54) **GLUCOSE AND/OR FRUCTOSE TRANSPORTER NaGLT1 AND ITS GENE**

(30) Priority: 13.12.2002 JP 2002363014
(71) Applicant: Japan Science and Technology Agency, Saitama 332-0012 (JP)
(72) Inventor: INUI, Ken-ichi Residence No. 3-43, Sakyo-ku, Kyoto-shi, Kyoto 606-8107 (JP); MASUDA, Satohiro, Sakyo-ku, Kyoto-shi, Kyoto 606-0931 (JP)
(74) Representative: Williams, Aylsa
(86) International application number: PCT/JP2003/015418
(87) International publication number: WO 2004/055184

(57) **Abstract**

The present invention provides a novel glucose and/or fructose transporter involved in renal diabetes, its gene, their variant, and their use. The glucose and/or fructose transporter of the present invention and its gene are a glucose and/or fructose transporter involved in renal diabetes and its gene, the protein and the gene are highly expressed in the kidney, and comprise a novel protein that make a larger contribution to the renal glucose reabsorption and its gene. The present invention comprises a variant of the protein and the gene, and an antibody that specifically binds to the protein. Further, the present invention comprises a non-human animal model wherein the gene of the present invention is deficient in its chromosome, a method for screening a preventive/therapeutic drug for renal diabetes, a method for diagnosing glucose and/or fructose transporter function and renal diseases using the gene or the antibody, and a method for regulating a transporter function in a tissue cell by introducing the gene of the present invention into the tissue cell.

## Description

### Technical Field

The present invention relates to a glucose and/or fructose transporter, its gene, its variant, and their use.

### Background Art

Although the weight of the human kidney, even in the total weight of two kidneys, is only 0.5% of the total body weight, about 20% of cardiac output, that is, 1 to 1.2 L of blood flows into the kidney every minute. About 20% of the blood plasma flow, in other words, 120 mL per minute (200 L per day) is filtered by the glomerulus and will be primary urine. 99% of the primary urine is reabsorbed in the renal tubule, and the daily urine output is the rest of the primary urine, which is 1.5 to 2 L. The kidney is constituted of nephrons, the functional unit, and vascular systems surrounding them. The nephron begins at the glomerulus, and reaches to the collecting duct system through the proximal tubule, the intermediate tubular region (thin limb of Henle's loop), and the distal tubule. Each segment has different function and configuration, and is involved in the concentration and the production of urine cooperatively. Particularly, in cells constituting the proximal tubule, the surface area is large due to the development of the brush border membrane of luminal side, and this is an efficient configuration for reabsorbing low-molecular nutritional substances as well as water and electrolytes in primary urine, and for secreting pharmaceuticals and foreign bodies in blood (The Pharmaceuticals Monthly, Vol. 43, No. 3, pp29-34, 2001; The Pharmaceuticals Monthly, Vol. 42, No. 4, pp113-120, 2000).

In the basolateral membrane of blood side and the brush border membrane of luminal side in epithelial cells of the proximal tubule, transporters which mediate the reabsorption and the secretion of various substances thus described are localized, and a network capable of direction-selective transportation of substances is formed (BIO Clinica, 11, 22-25, 1996; Seitai no Kagaku, 50, 268-273, 1999) . In the past several years, the cloning and the structural/functional analysis of a glucose transporter gene which regulates renal glucose excretion has been markedly advanced, and a mechanism in the kidney wherein glucose which has been transferred into urine in the glomerulus is reabsorbed and returned into blood is being elucidated at molecular level.

In other words, glucose in blood is filtered in the renal glomerulus, and reabsorbed in the renal tubule. By this mechanism, 100% of glucose is reabsorbed and returned into circulating blood when blood glucose level is normal.

Because water-soluble molecules such as glucose and amino acids, and ions cannot pass through biomembranes comprising a phospholipid bilayer quickly, transporter proteins which specifically transport these molecules generally exist in cell membranes and membrane organelles. A membrane protein which transports substances intracellulary and extracellulary is a transporter, and such transporters are involved in the process of transportation of pharmaceuticals, nutrients, etc., which has been taken into living bodies, to each tissue, and the excretion of wastes accumulated in each tissue (Japanese Laid-Open Patent Application No. 2002-171980). In the kidney, transporters (transport proteins) which mediate the reabsorption and secretion of various substances are localized on the basolateral membrane of blood side and the brush border membrane of luminal side, in epithelial cells of the proximal tubule, and a network capable of direction-selective transportation of substances, which takes advantage of environmental characteristics such as the difference in membrane potentials or pH gradient generated between inside and outside of cells, is formed (BIO Clinica, 11, 22-25, 1996; Seitai no Kagaku, 50, 268-273, 1999).

Glucose transporters of vertebrates are roughly classified into two types. One is called the facilitated glucose transporter: GLUT, and is a transport carrier of facilitate diffusion type which transports according to glucose concentration gradient generated between inside and outside of cells. Further, there are eight isoforms for this protein, the 12-transmembrane protein whose molecular weight is about 50,000 (Annu. Rev. Physiol. 55, 591-608, 1993; TIBS 23, 476-481, 1998; Japanese Laid-Open Patent Application No. 2002-218981). Basically, all cells express at least one type of GLUT, thereby obtaining necessary glucose extracellularly. The other is called the Na⁺-glucose cotransporter: SGLT, an active transport carrier which transports glucose against the concentration gradient by conjugating Na ion, and is a 14-transmembrane protein whose molecular weight is about 75,000.

This protein is present on the apical membrane of epithelial cells faced to the luminal side of the small intestine and the kidney, and conducts active transport wherein glucose is taken into cells with the use of gradient of electrochemical potential of Na⁺ generated between inside and outside of cells (Physiol. Rev. 74, 993-1026, 1994; Am. J. Physiol. 276, (5 Pt 1), G1251-1259, 1999; Japanese Laid-Open Patent Application No. 2002-218981). Glucose and Na⁺ taken into epithelial cells by SGLT are released into blood by the action of Na⁺-K⁺ pump and GLUT-2 present on the basolateral membrane.

SGLTs of mammals are further classified into two types, SGLT1 and SGLT2, according to their transport property. SGLT1 transports two Na⁺ ions per one molecule of sugar, has high affinity to glucose and galactose, and is expressed in the small intestine and the kidney. On the other hand, SGLT2 transports one Na⁺ ion per one molecule of sugar, has low affinity to glucose, and does not transport galactose. This protein is expressed in the kidney, however, its expression is not observed in the small intestine. In addition, SGLT1 and SGLT2 function in different sites in the kidney. Most of glucose transferred into urine in the glomerulus is first reabsorbed by SGLT2 in the proximal tubule, and further, completely reabsorbed by SGLT1 in the distal tubule, and returned into blood. By contrast, all glucose in food is absorbed into the body by SGLT1 in the small intestine.

As mentioned above, glucose transporters SGLT1 and SGLT2 are expressed in the kidney, and it is considered that the reabsorption process of glucose, which has been filtered in the glomerulus, into epithelial cells of renal tubules is mediated by these two transporters.

On the other hand, there is a condition called renal diabetes, wherein glycosuria is clearly observed even though glucose concentration in plasma is in the normal range (170 mg/dL or less). It is most often the case that the maximum rate (shown by TmG) that glucose which has been passed through the glomerulus is reabsorbed in the renal tubule, which is 350 mg/min for normal persons, is abnormally low. As a result, the glucose concentration in urine becomes abnormally high irrespective of the glucose concentration in plasma. This phenomenon is also observed in cases of abnormal proximal tubule, in other words, congenital or acquired Fanconi syndrome, or after phloridzin injections. As another cause of renal diabetes, there is a case wherein apparent threshold of glucose is lowered but both mean value of threshold and Tmg are perfectly normal. Abnormal increase in the excretion of glucose into urine found in such case is found only when the glucose concentration in plasma is low. Glucose excretion at plasma level exceeding the maximum threshold is rather normal (Medical Dictionary, 18^{th} Ed., pp1059-1060, Nanzando Co., Ltd., 1998; Biochemical Dictionary, 2^{nd} Ed., pp673, Tokyo Kagaku Dozin Co., Ltd., 1990).

Renal diabetes is thought to be caused by the a defect in renal glucose reabsorption, and the defect in glucose reabsorption is thought to be caused by congenital or acquired deficiency of known two types of transporters, in other words, SGLT1 and SGLT2, which are considered to mediate reabsorption process of glucose filtered in the glomerulus into epithelial cells of the renal tubule in the kidney. However, its causal gene has not been identified yet, and it has been considered to be caused by congenital or acquired deficiency of the known SGLT1 and SGLT2 genes, and other unknown transporter genes.

The development of pharmaceuticals for targeting or avoiding renal diseases is important to conduct drug therapy for renal diseases more efficiently and safely, however, there is no efficient successful examples in the present circumstances due to the lack of the past technical study basis for elucidating the cause of renal diseases.

The object of the present invention is to provide a novel glucose transporter which is expressed in the kidney and involved in renal glucose reabsorption, its gene, their variants, and their use.

It is thought that renal diabetes is caused by a defect in renal glucose reabsorption, and that the defect is caused by congenital or acquired def iciency of known glucose transporter genes, SGLT1 and SGLT2, however, its causal gene has not been identified yet. The present inventors have conducted intensive search for the unidentified gene, and have found a gene, other than the known SGLT1 and SGLT2 glucose transporter genes, which is highly expressed in the kidney, and the present invention has been completed.

The glucose transporter of the present invention shows higher amount of expression than that of the known SGLT1 and SGLT2, and makes a larger contribution to the renal glucose reabsorption, while it has a property to recognize glucose specifically. The glucose transporter of the present invention is designated as "glucose transporter NaGLT1". In addition, as a result of study, it is found that NaGLT1 of the present invention has Na⁺-dependent fructose transporter function.

The present invention also comprises a glucose and/or fructose transporter NaGLT1 and a variant of its gene, and an antibody that specifically binds to the glucose and/or fructose transporter NaGLT1. The present invention further comprises a non-human animal model which develops renal diabetes, whose gene function to express a polypeptide which has glucose and/or fructose transporter function of the present invention is deficient in its chromosome, and the screening of a preventive/therapeutic drug for renal diabetes with the use of the non-human animal model, and moreover , a method for diagnosing glucose and/or fructose transporter function and renal diseases with the use of the gene and the antibody of the present invention and a diagnostic drug for the method. The present invention still further comprises a method for regulating glucose and/or fructose transporter function in an animal tissue cell by controlling the expression of the gene of the present invention with the use of an antisense strand DNA, etc.

### Disclosure of the Invention

The present invention specifically comprise; a DNA which comprises a base sequence shown by SEQ ID NO: 1 in the sequence listing or its complementary sequence, or a sequence containing part or whole of these sequences ("1"), a DNA which hybridizes with the DNA according to "1" under a stringent condition, and which encodes a polypeptide having glucose and/or fructose transporter function ("2"), a DNA which encodes the following polypeptide (a) or (b); (a) a polypeptide which comprises an amino acid sequence shown by SEQ ID NO: 2 in the sequence listing, (b) a polypeptide which comprises an amino acid sequence wherein one or a few amino acids are deleted, substituted or added in the amino acid sequence shown by SEQ ID NO: 2 in the sequence listing, and which has glucose and/or fructose transporter function ("3"), a polypeptide which comprises an amino acid sequence shown by SEQ ID NO: 2 in the sequence listing ("4"), a polypeptide which comprises an amino acid sequence wherein one or a few amino acids are deleted, substituted or added in the amino acid sequence shown by SEQ ID NO: 2 in the sequence listing, and which has glucose and/or fructose transporter function ("5"), a method for producing a polypeptide which has glucose and/or fructose transporter function, wherein the DNA according to "1" to "3" is incorporated into an expression vector and expressed by introducing the recombinant expression vector into a host cell ("6"), an antibody which is induced by using the polypeptide according to "4" or "5" , and which binds to the polypeptide specifically ("7"), the antibody according to "7", wherein the antibody is a monoclonal antibody ("8"), the antibody according to "7", wherein the antibody is a polyclonal antibody ("9") .

The present invention also comprises; a method for producing an animal tissue cell expressing a polypeptide which has glucose and/or fructose transporter function, wherein the DNA according to any one of "1" to "3" is introduced into an animal tissue cell (''10''), the method for producing an animal tissue cell expressing a polypeptide which has glucose and/or fructose transporter function according to "10", wherein the animal tissue cell is a tissue cell of rat kidney, an epithelial cell derived from porcine kidney, an epithelial cell derived from canine kidney or an epithelial cell derived from opossum kidney ("11"), the method for producing an animal tissue cell expressing a polypeptide which has glucose and/or fructose transporter function according to "10'', wherein the animal tissue cell is HEK293, a transfected human embryonic kidney cell line ("12"), an animal tissue cell expressing a polypeptide which has glucose and/or fructose transporter function, which is produced by the method according to any one of "10" to "12" ("13"), a method for screening a substance having a glucose and/or fructose transporter function-regulating activity, wherein an effect of a test substance on glucose transport function is measured with the use of the animal tissue cell expressing a polypeptide which has glucose and/or fructose transporter function according to "13" ("14"), a non-human animal model which develops renal diabetes caused by a defect in renal glucose reabsorption, whose gene function to express a polypeptide which has glucose and/or fructose transporter function shown by SEQ ID NO: 2 in the sequence listing is deficient in its chromosome ("15"), the non-human animal model which develops renal diabetes according to "15", wherein the deficiency in the gene function to express a polypeptide which has glucose and/or fructose transporter function is deficiency in a function of a gene which expresses a polypeptide which has glucose and/or fructose transporter function shown by SEQ ID NO: 1 in the sequence listing ("16"), a method for screening a preventive/therapeutic drug for renal diabetes caused by a defect in glucose reabsorption, wherein a test substance is administered to the non-human animal model which develops renal diabetes caused by a defect in renal glucose and/or fructose reabsorption according to "15" or "16", and glucose reabsorption ability of the non-human animal model, or a cell, a tissue or an organ of the non-human animal model is measured/evaluated ("17"), a probe for diagnosing glucose and/or fructose transporter function comprising whole or part of an antisense strand of the base sequence according to "1" ("18"), a microarray or a DNA chip for diagnosing glucose and/or fructose transporter function, wherein at least one DNA according to any one of "1" to "3" is immobilized ("19").

The present invention further comprises; a pharmaceutical for diagnosing glucose and/or fructose transporter function, wherein the antibody according to any one of "7" to "9" and/or the probe for diagnosing according to "18" is prepared ("20"), a method for diagnosing glucose and/or fructose transporter function, wherein a sample is obtained from a test substance, and the expression of the gene according to "1" in the sample is measured ("21"), a method for diagnosing glucose and/or fructose transporter function, wherein the measurement of the gene expression according to "21" is conducted with the probe for diagnosing glucose and/or fructose transporter function according to "18", or with the microarray or the DNA chip for diagnosing glucose and/or fructose transporter function according to "19" ("22"), a method for diagnosing glucose and/or fructose transporter function, wherein a sample is obtained from a test substance and cultured, and the polypeptide according to "4" produced by the expression of the gene in the sample is measured ("23"), a method for diagnosing glucose and/or fructose transporter function, wherein the measurement of the polypeptide according to "23" is conducted with the antibody according to any one of "7" to "9" ("24"), a method for diagnosing a renal disease, wherein the diagnosis of glucose and/or fructose transporter function according to any one of "21" to "24" is measurement of glucose and/or fructose transporter function in a renal disease ("25"), a method for regulating glucose and/or fructose transporter function in an animal tissue cell, wherein the DNA according to any one of "1" to "3" is introduced into an animal tissue cell ("26"), a method for regulating glucose and/or fructose transporter function in an animal tissue cell, wherein the expression of the DNA according to "1" is suppressed in an animal tissue cell ("27"), a method for regulating glucose and/or fructose transporter function in an animal tissue cell, wherein the expression of the DNA according to "1" is suppressed in an animal tissue cell by introducing whole or part of an antisense strand of the DNA base sequence according to "1" into an animal tissue cell ("28"), the method for regulating glucose and/or fructose transporter function in an animal tissue cell according to any one of "26" to "28", wherein the animal tissue cell is an animal kidney cell ("29").

### Brief Description of Drawings

Fig. 1: (A) shows the base and amino acid sequences of NaGLT1 of the present invention, and (B) shows hydropathy plot (hydrophobicity analysis) of the amino acid sequence of NaGLT1 of the present invention, in Example of the present invention.
Fig. 2: (A) is a photograph showing Northern blot analysis of NaGLT1 mRNA in rat cells of the present invention, and (B) is a photograph showing that NaGLT1 mRNA in rat cells of the present invention was detected by PCR, in Example of the present invention.
Fig. 3 is a photograph showing renal expression distribution of each mRNA of NaGLT1, SGLT1, SGLT2, and GAPDH of the present invention, detected by PCR, in Example of the present invention. In the figure, (+) shows the case wherein RT-PCR was conducted under the condition that reverse transcriptase was contained, and (-) shows the case wherein RT-PCR was conducted under the condition that reverse transcriptase was not contained, respectively.
Fig. 4 shows that the expression amount of NaGLT1, SGLT1, and SGLT2 mRNA of the present invention was analyzed quantitatively by real-time PCR, in Example of the present invention.
Fig. 5 shows the accumulation of various saccharides in oocytes into which NaGLTl mRNA of the present invention which had been synthesized in vitro was injected, and in water-injected oocytes, in Example of the present invention.
Fig. 6: (A) shows the interdependence between accumulation amount of [¹⁴C]αMeGlc and αMeGlc itself in NaGLTl-expressing oocytes of the present invention, (B) shows Hill plot using the data of (A), (C) shows the interdependence between accumulation amount of [¹⁴C]αMeGlc and extracellular Na⁺ ion concentration, and (D) shows Hill plot using the data of (C), in Example of the present invention.
Fig. 7 shows the effect of various saccharides on accumulation of [¹⁴C]αMeGlc in NaGLT1-expressing oocytes and water-injected oocytes of the present invention, in Example of the present invention.
Fig. 8 is a photograph showing the result of immunoblotting with which intracellular localization of NaGLT1 protein in rat renal membrane sites (renal crude membrane, brush border membrane, and basolateral membrane) was analyzed in Example of the present invention.
Fig. 9 is a graph showing the uptake of fructose by HEK293 cells mediated by NaGLT1 in Example of the present invention, and (A) shows the case of HEK293 cells incubated with buffer solution containing glucose analogs for 15 minutes at 37°C, and (B) shows the uptake of [¹⁴C]fructose by HEK293 cells transfected with NaGLT1 cDNA.
Fig. 10 is a graph showing the uptake of fructose by renal brush border membrane vesicles in Example of the present invention, and (A) shows the uptake in the case where membrane vesicles suspended in mannitol and HEPES were incubated with a substrate mixture containing mannitol and HEPES at 25°C, and (B) shows the Na⁺-dependent uptake of fructose in incubation buffer solution at various concentrations.

### Best Mode of Carrying Out the Invention

### (The gene of the present invention, a polypeptide encoded by the gene and its antibody)

The present invention comprises a glucose and/or fructose transporter NaGLT1 involved in the cause of renal diabetes, its causal gene, and their variants.

The glucose and/or fructose transporter NaGLT1 cDNA involved in the cause of renal diabetes of the present invention has a base sequence shown by SEQ ID NO: 1 in the sequence listing. In addition, the glucose and/or fructose transporter NaGLT1 protein encoded by the cDNA comprises a polypeptide comprising amino acid sequence shown by SEQ ID NO: 2 in the sequence listing. The cDNA of glucose and/or fructose transporter NaGLT1 , a novel gene obtained in the present invention, comprises 2,173 base pairs, and a polypeptide comprising 484 amino acid residues is encoded in its translation region (111^{th} to 1562^{nd} position).

It is confirmed that the glucose and/or fructose transporter NaGLT1 of the present invention is an 11-transmembrane glycoprotein according to the result of hydrophobicity analysis. The glucose and/or fructose transporter NaGLT1 of the present invention is a glucose and/or fructose transporter which shows high expression in the kidney, and recognizes not other monosaccharides but glucose and/or fructose specifically. Expression amount of the glucose and/or fructose transporter NaGLT1 in kidney is higher than that of known glucose transporters SGLT1 or SGLT2, making a great contribution to the renal glucose reabsorption, which is thought to be the cause of renal diabetes.

The gene of the present invention includes: the aforementioned base sequence shown by SEQ ID NO: 1 in the sequence listing or its complementary sequence, or a sequence containing part or whole of these sequences, further, a DNA sequence which hybridizes the base sequence under a stringent condition and which encodes a polypeptide having glucose and/or fructose transporter function, and a DNA which encodes the following polypeptide (a) or (b);
(a) a polypeptide which comprises an amino acid sequence shown by SEQ ID NO: 2 in the sequence listing,
(b) a polypeptide which comprises an amino acid sequence wherein one or a few amino acids are deleted, substituted or added in the amino acid sequence shown by SEQ ID NO: 2 in the sequence listing and which has a polypeptide having glucose and/or fructose transporter function.

In the present invention, various DNA sequence mutations can be conducted by known gene-mutating means of genetic engineering.

As to the base sequence of the present invention mentioned above, the condition, "to hybridize the base sequence under a stringent condition", is exemplified by hybridization at 42°C, and washing treatment at 42°C with buffer solution containing 1 x SSC, 0.1% SDS, and more preferably exemplified by hybridization at 65°C, and washing treatment at 65°C with buffer solution containing 0.1 x SSC, 0.1% SDS. There are various factors other than the temperature condition mentioned above that affect the hybridization stringency and those skilled in the art can actualize the same stringency as that of the hybridization referred to in the above by appropriately combining various factors.

Further, the present invention includes a polypeptide which comprises an amino acid sequence shown by SEQ ID NO: 2 in the sequence listing, a polypeptide which comprises an amino acid sequence wherein one or a few amino acids are deleted, substituted or added in the amino acid sequence shown by SEQ ID NO in the sequence listing and which has glucose and/or fructose transporter function. The polypeptide of the present invention can be obtained by known technology of genetic engineering. In other words, the polypeptide can be obtained by incorporating the gene of the present invention into a known expression vector appropriately, introducing the recombinant vector into a host cell, and expressing it.

### (Use of antibodies induced by the polypeptide of the present invention)

The present invention further includes an antibody induced by the polypeptide of the present invention and binding to the polypeptide specifically. As the antibody, a monoclonal antibody and a polyclonal antibody can be exemplified. The antibody can be produced by an ordinary method using the polypeptide of the present invention as an antigen. The antibody of the present invention can be used for detecting the expression of the gene of the present invention in renal tissue cells, etc., by an antigen-antibody reaction with the polypeptide of the present invention which has glucose and/or fructose transporter function, and for diagnosing renal diseases relating to the gene. As immunoassay using the antibody of the present invention, for example, known immunoassay such as RIA method, ELISA method and fluorescent antibody technique can be used.

### (Use of human tissue cells into which the DNA of the present invention is introduced)

Human tissue cells expressing a polypeptide which has glucose and/or fructose transporter function can be produced by introducing the DNA of the present invention into human tissue cells. As for the human tissue cells, it is preferable to use renal tissue cells originally expressing NaGLT1 strongly, and a specific example of the renal tissue cells is HEK293, a transfected human embryonic kidney cell line. In addition, as an animal tissue cell into which the gene of the present invention is introduced, any of the followings can be also used: a rat kidney tissue cell; an epithelial cell derived from porcine kidney, LLC- PK₁; an epithelial cell derived from canine kidney, MDCK; an epithelial cell derived from opossum kidney, OK. In order to introduce the DNA of the present invention in to human tissue cells, appropriate gene introduction methods such as transfection can be used.

### (Use of the gene of the present invention and a polypeptide encoded by the gene)

In the present invention, a novel gene of NaGLT1 , cloned from human renal tissues, can be used as a diagnostic probe for diagnosing glucose and/or fructose transporter function in renal tissue cells with the use of an antisense strand of the base sequence. Further, the gene can be used as a diagnostic drug for glucose and/or fructose transporter function with the use of the diagnostic probe and the antibodywhich specifically binds to the polypeptide of the present invention, and as a diagnostic kit containing the diagnostic drug.

In addition, at lease one DNA of the present invention can be fixed on a device and used as a microarray or a DNA chip for diagnosing glucose and/or fructose transporter function. Onto the microarray or the DNA chip, other genes of a glucose and/or fructose transporter, etc., can be fixed together, and used for diagnosis. Further, in order to measure an expression status of the gene of the present invention in renal tissue cells, known gene measurement methods such as RT-PCR and Northern blotting can be appropriately used. Genetic diseases in the human kidney can be detected by measuring the presence or the intensity of expression of the glucose and/or fructose transporter NaGLT1 gene in renal tissue cells, with the use of the diagnostic method of the present invention.

### (Regulation of glucose and/or fructose transporter function by using the gene of the present invention)

Glucose and/or fructose transporter function in human tissue cells can be regulated by controlling the expression of the gene of the present invention in human tissue cells. The gene expression in human tissue cells can be controlled by introducing the gene of the present invention, or an antisense strand for the gene of the present invention into human tissue cells to suppress the expression of the gene of the present invention. As a method for introducing the gene of the present invention into human tissue cells, known gene introduction methods such as transfection can be used. In order to introduce an antisense strand into human tissue cells, methods usually used in this field can be used. For example, it is possible to administer an antisense oligonucleotide directly into human tissue cells such as renal tissue. In addition, if necessary, it is possible to administer together with pharmaceutically acceptable intracellular introduction reagents, for instance, lipofectin reagent, lipofectamine reagent, DOTAP reagent, Tfx reagent, liposome and polymeric carriers, etc.

Genetic diseases in the kidney, etc., can be prevented/treated by regulating glucose and/or fructose transporter function in human tissue cells such as the kidney.

### (Gene-deficient non-human animal model of the present invention and its use)

The non-human animal model which develops renal diabetes of the present invention means a non-human animal which develops renal diabetes such as a defect in renal glucose and/or fructose reabsorption caused by deficiency in the function of the glucose and/or fructose transporter NaGLT1 gene in its chromosome. Specific examples of the non-human animal of the present invention include rodents such as a rat, a mouse, a guinea pig, and as a non-human animal model used for the screening of preventive/therapeutic drug for renal diabetes of the present invention, a mouse and a rat can be used particularly advantageously, but such animals is not limited thereto.

The non-human animal model of the present invention whose function of NaGLT1 gene is deficient in its chromosome can be produced by known methods for producing non-human animal models whose gene is deficient. The method for producing non-human animal models whose function of NaGLT1 gene is deficient in its chromosome is described below with reference to a mouse whose function of NaGLT1 gene is deficient in its chromosome as an example.

A mouse whose function of NaGLT1 gene is deficient in its chromosome, in other words, a homozygous mutant mouse (-/-) can be constructed, for example, by the method comprising the steps of: a NaGLT1 gene is screened by using a gene fragment obtained from rat gene library constructed from rat kidney; part or whole of the screened NaGLT1 gene is substituted with a marker gene, for example, a lac-Z gene, a neomycin-resistant gene or the like, and if necessary, a gene such as a diphthelia toxin A fragment (DT-A) gene or a herpes simplex virus thymidine kinase (HSV-tk) gene is introduced into 5'-terminal side, to construct a targeting vector; this constructed targeting vector is linearized and introduced into an ES cell by electroporation or other such methods and then homologous recombination is conducted; among the homologous recombinants, an ES cell resistant to X-gal staining or antibiotics such as G418, ganciclovir (GANC) is selected. It is preferable to confirm whether the selected ES cell is the intended recombinant by Southern blotting or other such methods.

The above-mentioned recombined ES cell is microinjected into a mouse blastocyst, and then the blastocyst is transplanted into a recipient mouse to generate a chimeric mouse. A heterozygous mutant mouse (+/-) can be obtained by intercrossing the chimeric mouse and a wild-type mouse, and a homozygous mutant mouse (-/-) can be obtained by intercrossing the heterozygous mutant mice. As examples of the method for confirming whether NaGLT1 gene is deficient in the homozygous mutant mouse include Western blotting or other such methods with which the expression of NaGLT1 gene in the mouse is confirmed.

The screening of a preventive/therapeutic drug for renal diabetes caused by a defect in renal glucose reabsorption of the present invention is conducted by administering a test substance to the non-human animal which develops renal diabetes such as a defect in renal glucose and/or fructose reabsorption caused by deficiency in the function of the glucose and/or fructose transporter NaGLT1 gene in its chromosome, and by measuring/evaluating glucose and/or fructose reabsorption ability of the non-human animal model, or a cell, a tissue or an organ of the non-human animal model.

As mentioned above, when screening a preventive/ therapeutic drug for renal diabetes caused by a defect in renal glucose and/or fructose reabsorption , it is preferable to compare/evaluate with the case of the non-human animal which develops renal diabetes caused by a defect in glucose and/or fructose reabsorption with the use of a wild-type non-human animal and/or a non-human animal whose function of NaGLT1 gene is deficient in its chromosome. The wild-type non-human animal means a wild-type non-human animal which is the same species as the non-human animal whose function of NaGLT1 gene is deficient in its chromosome, and the littermate thereof is more preferably exemplified. The non-human animal whose function of NaGLT1 gene is deficient in its chromosome can be produced by the method described previously (Proc. Natl. Acad. Sci. USA 97, 6132-6137, 2000), etc. It is preferable to use a type whose function of NaGLT1 gene is deficient and its wild-type littermate simultaneously because precise comparative experiments, evaluation (analysis) etc . , can be conducted at an individual level.

### (Regulation of glucose and/or fructose transporter function using the gene of the present invention)

Glucose and/or fructose transporter function in animal tissue cells can be regulated by controlling the expression of the gene of the present invention in animal tissue cells, in particular, renal tissue cells. The gene expression in animal tissue cells can be controlled by introducing the gene of the present invention, or an antisense strand and siRNA for the gene of the present invention into animal tissue cells to suppress the expression of the gene of the present invention. As an introduction method of the gene of the present invention into animal tissue cells, known gene introduction method such as transfection can be used. In order to introduce antisense strands into animal tissue cells, methods usually used in this field can be used. For example, it is possible to administer an antisense oligonucleotide directly into animal tissue cells such as renal tissue. In addition, if necessary, it is possible to administer together with pharmaceutically acceptable intracellular introduction reagents, for instance, lipofectin reagent, lipofectamine reagent, DOTAP reagent, Tfx reagent, liposome and polymeric carrier, etc.

Genetic diseases in renal diabetes, etc., can be prevented/treated by regulating glucose and/or fructose transporter function in animal tissue cells such as kidney.

The present invention is described below more specifically with reference to Examples, however, the technical scope of the present invention is not limited to these exemplification.

### [Example]

### (cDNA cloning of the glucose and/or fructose transporter NaGLT1)

Total RNA was extracted from rat kidney by cesium chloride density-gradient centrifugation, and poly A + RNA (mRNA) was purified from the total RNA with oligo dT cellurose (Stratagene). Based on the purified mRNA, rat kidney cDNA library was constructed with cDNA library constructing kit (Stratagene). From the cDNA library, 1,000 genes were picked up at random, and sequencing was conducted with a vector primer (T3 primer; 5'-AATTAACCCTCACTAAAGGG-3'). As for full-length sequencing of NaGLT1, a primer (SEQ ID NOs: 3 to 12 in the sequence listing) was designed as described in Table 1 mentioned below (custom synthesized by Proligo), decoding was conducted by chain terminator method by using RISA-384 (Shimadzu Corporation), and the sequence listing was constructed with GENETYX-MAC Version 10 (SOFTWARE DEVELOPMENT, Tokyo). Actual sequencing was conducted by RISA-384 system (contracting analysis to Shimadzu Corporation, Genomic Research Laboratory).

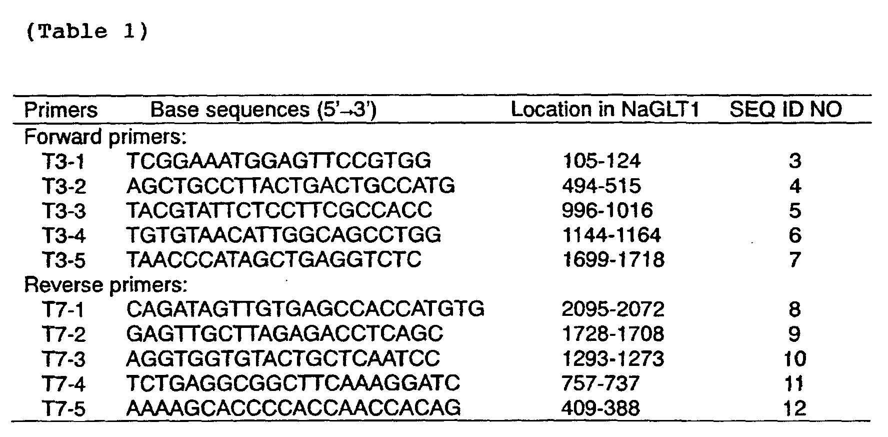

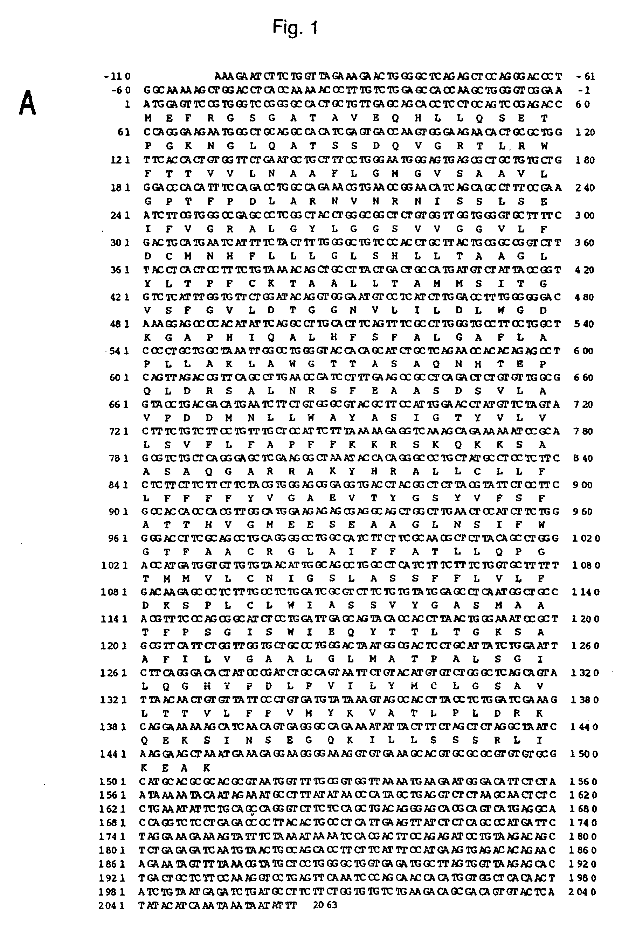

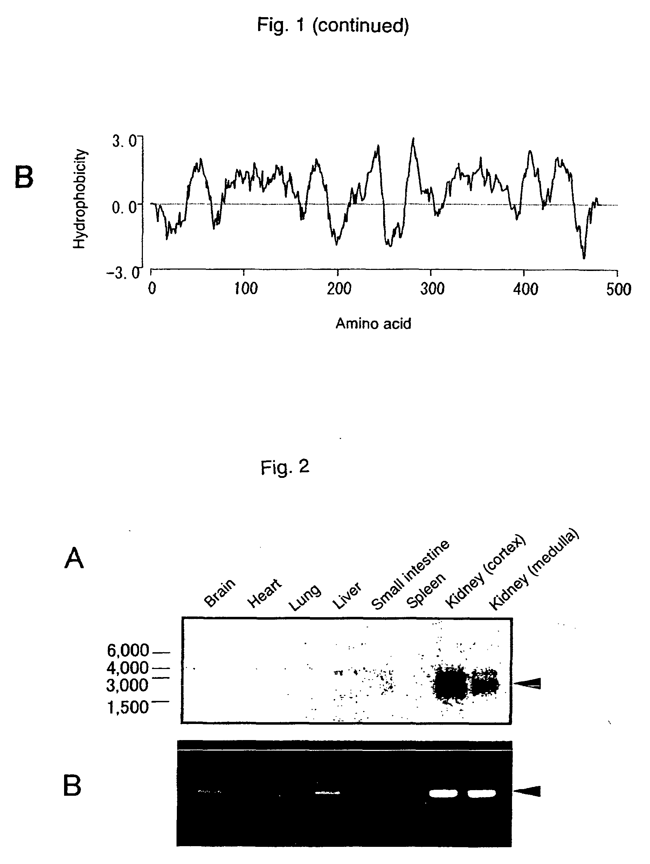

Homology analysis was conducted between the obtained gene sequence information and the gene sequences registered in each of data bases, GenBank, EMBL, DDBJ and PDB, by using BLAST, and the information was classified into a known group and an unknown group. In regard to about 200 kinds of unknown genes, their mRNAs were synthesized in vitro with mCap RNA Capping kit (Stratagene), and a transport experiment was conducted with the expression system of Xenopus oocytes. As a result, a clone (NaGLT1) which specifically transports metabolism-resistant α-methyl-D-glucopyranoside (αMeGlc) was identified (GenBank accession No: AB089802). The isolated NaGLT1 cDNA comprised 2,173 base pairs (SEQ ID NO: 1) and a protein comprising 484 amino acid residues (SEQ ID NO: 2) was encoded in its translation region (111^{th} to 1562^{nd} position) (Fig. 1A). It was presumed that NaGLT1 would be an 11-transmembrane glycoprotein according to the result of hydorphobicity analysis (Fig. 1B).

### (Analysis of distribution of NaGLT1 expression by Northern blotting and RT-PCR)

Under the stringent condition [50% formamide, 5 x SSPE (composition of 1 × SSPE: 0.15 M NaCl, 10 mM NaH₂PO₄, and 1 mM EDTA; pH 7.4), 5 × Denhardt ' s solution, 0.2% SDS , and 10 µm/ml DNA derived from herring sperm, at 42°C], cDNA (full length) of NaGLT1 was labeled with [α⁻³²P] dCTP (Amersham) by using Prime-a-Gene Labeling System (Promega), and total RNA was extracted from each organ of rat with RNeasy mini RNA extraction kit (QIAGEN) and was hybridized to a blotted nylon membrane (Hybond N+ membrane: Amersham). After the hybridization, blotted membrane was washed twice with 2 x SSC (composition of 1 x SSC: 0.15 M NaCl, 15 mM sodium citrate, pH 7.0)/0.1% SDS for 10 minutes at room temperature, and further washed once with 0.5 x SSC/0.1% SDS for 30 minutes at 42°C. The blotted membrane thus washed was exposed to an imaging plate for 3 to 6 hours, visualized bands were read with an imaging plate reader, and subsequently, image processing was conducted with Image Analyze II (Fuji Photo Film Co., Ltd.) (BIO-imaging Analyzer BAS-2000II system, Fuji Photo Film Co., Ltd.) . The value quantification was conducted for the intensity of radiation corresponding to each band on Image Analyze II. As a result, it was observed that NaGLT1 mRNA was highly expressed in kidney cortex and medulla (Fig. 2A).

Then, for the examination by RT-PCR, 1 pg of total RNA was obtained from each tissue of rat (brain, heart, lung, liver, small intestine, spleen, kidney cortex, kidney medulla) with RNeasy mini kit (QIAGEN), and reverse transcription reaction was conducted with SuperScript II reverse transcriptase (Invitrogen). After the reaction, remained RNA was digested with RNase H (Invitrogen). Heat denaturation was conducted for 3 minutes at 95°C with the obtained single-stranded DNA as a template by using primers specific to NaGLT1 , SGLT1, SGLT2 and GAPDH (see Table 2 below and SEQ ID NOs : 13 to 20 in the sequence listing), and Taq DNA polymerase (Takara), then the following cycle was repeated 35 times for amplification. Cycle: heat denaturation for 1 minute at 94°C, annealing for 1 minute at 58°C, and elongation reaction for 1 minute at 72°C. RT-PCR product obtained with the cycle was separated on 1.5% agarose gel, and subsequently stained with ethidium bromide and visualized under UV rays. As a result, it was observed that NaGLT1 was highly expressed in the kidney (cortex, medulla), and other than the kidney, expression over detection limit was observed in the brain, the lung and the liver (Fig. 2B).

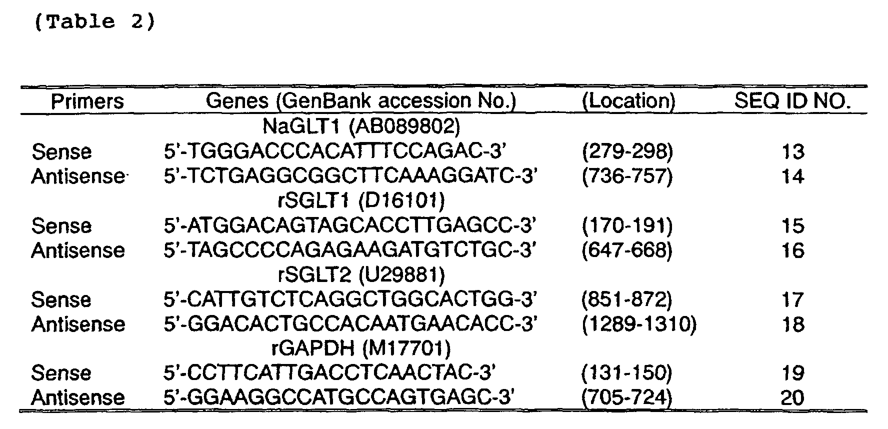

### (Isolation of rat renal tubule segments by microdissection)

Male Wistar rat (7 weeks old) was subjected to midline laparotomy under Nembutal anesthesia to expose the left kidney. Cranial side just before the diverging point of left renal artery in inferior aorta was ligated. Cranial side just before the lateral diverging point of inferior aorta and inferior vena cava was ligated, and a small hole was made in a blood vessel at caudal side, immediately under the left renal artery. Subsequently, polyethylene medical tube (PE-50, Becton Dickinson) was inserted through the small hole to preperfuse the left kidney with 10 ml of solution A (130 mM NaCl , 5 mM KCl , 1 mM NaH₂PO₄, 1 mM magnesium sulfate, 1 mM calcium lactate, 2 mM sodium acetate, 5.5 mM D-glucose, 10 mM HEPES, pH 7.4) by using a 10 ml syringe without putting on overpressure. After confirming that no congestion, etc. , was observed in the perfused kidney, the kidney was perfused with 10 ml of solution B (solution A containing 1 mg/ml collagenase (type I, Sigma), 1 mg/ml bovine serum albumin (BSA) (Sigma), 10 mM vanadyl-ribonucleoside complex (Invitrogen)), and was quickly removed.

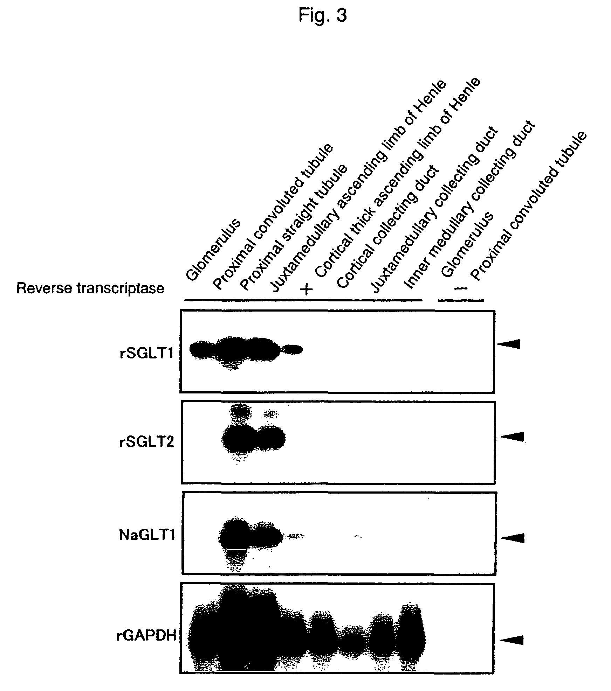

Renal section of 1 to 1.5 mm thick was prepared by cutting the kidney at an angle that covers the cortex and the medulla. The obtained renal section was oxygenized with 100% O₂ while being shaken in solution B at 37°C for 30 minutes. Then, the renal section was washed with ice-cold solution A, and each segment of renal tubule mentioned below was separated with a siliconized sharp needle while microscope observation was conducted based on each structural feature. As to each nephron segment thus isolated (glomerulus, proximal convoluted tubule, proximal straight tubule, medullary thick ascending limb of Henle, cortical thick ascending limb of Henle , cortical collecting duct, outer medullary collecting duct, inner medullary collecting duct), 20 glomeruli, and 8 mm of other segments were used as one sample, and total RNA was extracted from each segment sample with RNeasy RNA mini kit (QIAGEN). RT-PCR was conducted with the use of the obtained total RNA and the primer sets (SEQ ID NOs : 21 to 29) in Table 3 below (Fig. 3). As a result, it has been revealed that NaGLT1 mRNA is highly expressed in the proximal convoluted tubule and the proximal straight tubule, like other SGLTs (Fig. 3).

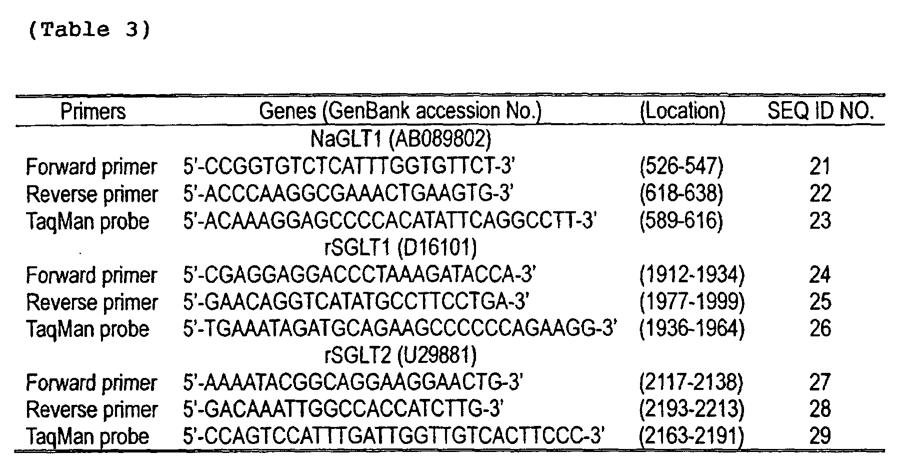

### (Quantitative analysis of NaGLT1 mRNA expression level)

Total RNA derived from the kidney of male Wistar rat (7 weeks old) was reverse-transcribed (see method 2), with the obtained single-stranded DNA as a template, the expression amounts of NaGLT1 , SGLT1, SGLT2 and GAPDH mRNAs were quantitated by using primers specific to NaGLT1, SGLT1, SGLT2 and GAPDH and TaqMan probes (see Table 3) and Universal master mix (Applied Biosystems), and with ABI PRISM 7700 Sequence Detection System. In order to obtain a standard curve, PCR product amplified by real-time PCR was inserted into pGEM-T Easy vector (Promega), and transfected to E. coli (DH-5α). The transfected E. coli was shaking-cultured overnight in LB broth (10 g bactotryptone, 5 g yeast extract, and 10 g NaCl in 1 L, pH 7.2), and plasmid DNA encoding amplified product (PCR fragments amplified with the primer sets in Table 2) of NaGLT1, SGLT1, SGLT2 and GAPDH, respectively, was purified.

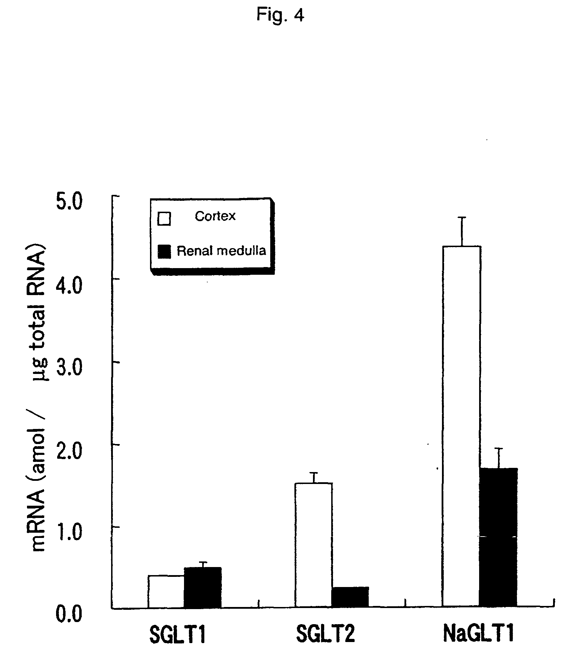

Each concentration was measured with a UV 1200 spectrophotometer (Shimadzu Corporation), and used as control gene at known concentration. Results obtained with PRISM 7700 were numerically converted with a standard curve. The expression amount of GAPDH used as an internal standard was used for adjusting the amount of template RNA in each reaction of real-time PCR. As a result, the expression amount of NaGLT1 mRNA was indicated to be higher than other SGLTs in both kidney cortex and medulla (Fig. 4).

### (Transport substrate of NaGLT1)

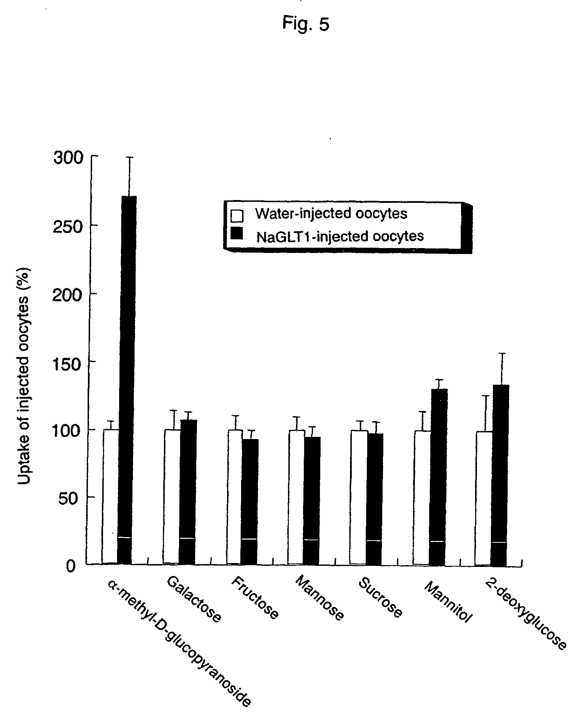

Transportation activity of NaGLT1 was examined with the expression system of Xenopus oocytes (hereinafter abbreviated as oocytes). First, in order to examine the selectivity of various saccharides, NaGLT1 RNA synthesized in vitro was injected into the oocyte and incubated for 2 days at 18°C, then used for the experiment. As a result of examination of Radiolabeled monosaccharide (α-methyl-D-glucopyranoside (αMeGlc: metabolism-resistant glucose), galactose, fructose, mannose, mannitol and 2-deoxyglucose) and disaccharide (sucrose) as a substrate, uptake of α-MeGlc only was significantly high in comparison to water-injected oocytes examined at the same time as a negative control (Fig. 5) . Therefore, it was revealed that the transport substrate of NaGLT1 is glucose.

### (Property of αMeGlc transportation mediated by NaGLT1)

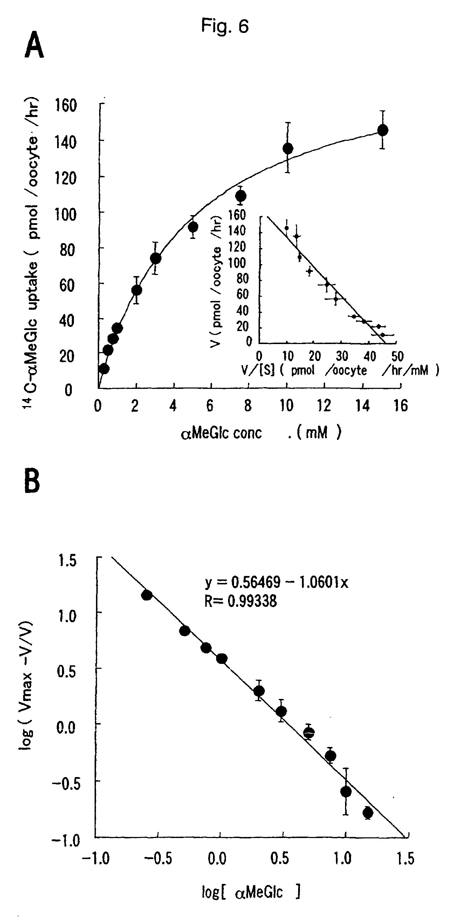

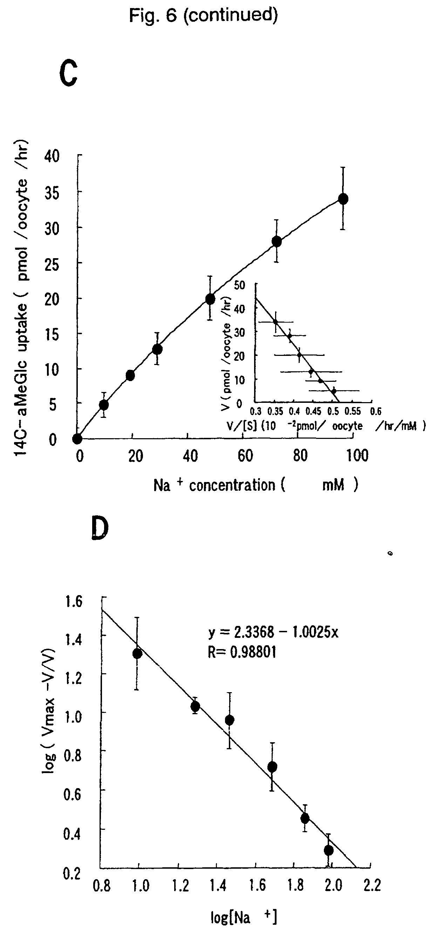

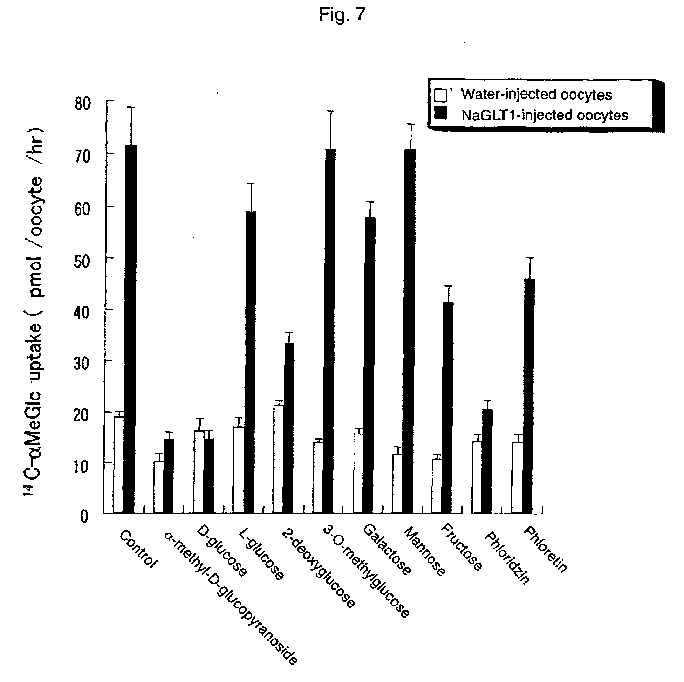

Uptake activity of NaGLT1 cRNA-injected cell and water-injected oocyte to [U-¹⁴C]-α-methyl-D-glucopyranoside (α-MeGlc), D-[1-¹⁴C] galactose, D-[U-¹⁴C] mannose, D-[U-¹⁴C] fructose, [1,2-³H]-2-deoxy-glucose, D-[1-³H] mannitol, [U-¹⁴C] sucrose was measured. At that time, incubation was conducted in a buffer solution comprising 96 mM NaCl, 2 mM KCl, 1.8 mM CaCl₂, 1 mM MgCl₂, 5 mM HEPES (pH 7.4). Further, when extracellular Na⁺ concentration dependency was conducted, the concentration of NaCl was adjusted to be within the range of 9.6 to 96 mM, and the final osmotic pressure was kept constant by making up for the shortage to 96 mM with choline chloride (Figs. 6 and 7).

Next, concentration dependency of αMeGlc uptake was examined by using NaGLT1-expressing oocytes. As a result, Km value was 3.71 ± 0.09 mM (Fig. 6A). In addition, the effect of extracellular Na⁺ ion concentration on the uptake of αMeGlc mediated by NaGLT1 was analyzed. First, extracellular Na⁺ concentration was varied from 0 to 96 mM, at the state wherein membrane potential difference was eliminated by the addition of 7 µM valinomycin, and the effect of extracellular Na⁺ on the uptake of αMeGlc by rNaGLT1 was examined. Uptake of αMeGlc increased in a manner dependent to extracellular Na⁺ concentration in cells into which rNaGLT1 RNA synthesized in vitro was injected. Further, by examination of dose- or extracellular Na⁺-dependancy to the uptake of αMeGlc by rNaGLT1 (Figs. 6A and 6C), Vmax value (the maximum transport rate) and Km value were calculated for each case. Based on those values, Hill plot (logarithmic values of the figure calculated by dividing Vmax by transport rate V when the abscissa axis is the concentration of varied substrate or ion, and ordinate axis is each substrate (or ion) concentration) was conducted (Figs. 6B and 6D) and Hill coefficient was calculated. As a result, Hill coefficient of αMeGlc and Na⁺ were 1.06 and 1.00, respectively, and coupling ratio of αMeGlc and Na⁺ was indicated to be 1:1 (Fig. 6). Therefore, it was suggested that NaGLT1 is a glucose transporter which functions in a manner dependent to extracellular Na⁺ ion concentration.

Further, the effect of various inhibitors on the uptake of [¹⁴C]-labeled αMeGlc mediated by NaGLT1-expressing oocytes was examined, and as a result, unlabeled αMeGlc, D-glucose, 2-deoxy glucose and phloridzin had an extremely strong inhibitory effect. Fructose and phloretin had a weak inhibitory effect. On the other hand, L-glucose, 3-O-methylglucose, galactose and mannose exhibited no effect on the uptake of αMeGlc mediated by NaGLT1 (Fig. 7).

### (Construction of anti-NaGLT1 antibody)

Based on the amino acid sequence of NaGLT1, a peptide at C-terminal side (H2N-LPLDRKQEKSINSEGQ-COOH) (SEQ ID NO: 30) was constructed with its N-terminal side as cysteine (custom synthesized by Sawady Technology Co., Ltd.). As a result of analysis with high-performance liquid chromatography (HPCL), the purity of the synthesized peptide was 92%. Then, a conjugate to this peptide was constructed by using hemocyanin (keyhole limpet hemocyanin (Calbiochem-Behring)). The conjugate was dispensed into 10 containers by 1 ml each, and stored in a frozen state.

As for the conjugate, uniform emulsion was prepared with Freund complete adjuvant (Difco). After collecting preimmunized serum of male Japanese white rabbit (2 kg), immunization was conducted at 0.2 mg/rabbit at 2-week intervals. Blood was collected at each immunization, and antibody titer was analyzed by ELISA method. After obtaining sufficient antibody titer eventually, whole blood was collected and stored in a frozen state as an anti-serum.

### (Localization analysis of NaGLT1 by immunoblotting)

Each tissue was extracted from male Wistar rat (220 to 230 g) under pentobarbital anesthesia, and homogenized with a homogenate buffer (230 mM sucrose, 5 mM Tris/HCl (pH 7.5), 2 m ethylenediamine tetra acetic acid (EDTA), 0.1 mM phenylmethylsulfonyl fluoride (PMSF)), and centrifugation was conducted for 15 minutes at 3000 g. The supernatant was removed and centrifugation was further conducted for 30 minutes at 24500 g to collect a precipitate (a crude memberane fraction). Rat renal brush border membrane and basolateral membrane were prepared simultaneously according to Percoll density-gradient centrifugation (Biochim Biophys Acta 773, 113-124, 1984). The membrane sample was solubilized to SDS sample buffer (2% SDS, 125 mM Tris, 20% glycerol), and polyacrylamide electrophoresis (Nature 227, 680-685, 1970) was conducted.

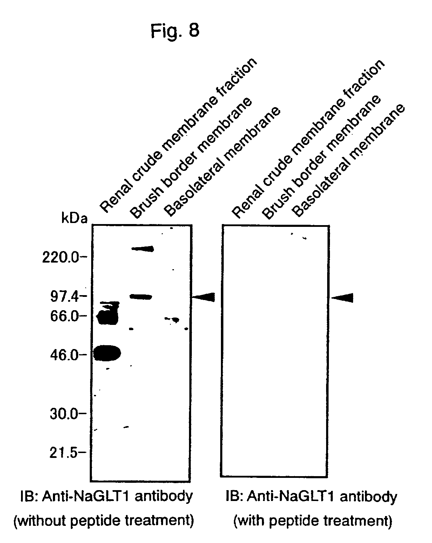

As a molecular weight marker, RainbowTM colored protein molecular weight markers [myosin (220,000), phosphorylase beta (97,400), BSA (66,000), ovalbumin (46,000), carbonic anhydrase (30,000), trypsin inhibitor (21,500), lysozyme (14,300)] (Amersham) were used. After the electrophoresis, the protein was transferred to PVDF membrane (Immobiron P, trtillipore), and then rinsed with TBS-T (Tris buffered saline; 20 mM Tris/HCl (pH 7.5), 137 mM NaCl, 0.1% Tween 20). Next, after blocking was conducted with TBS-T containing 5% BSA for 2 hours at room temperature, the membrane was made to react with anti-serum diluted with TBS-T containing 5% BSA (1:2000) at 4°C overnight, and then the membrane was washed three times with TBS-T for 15 minutes. By using ECL chemiluminescence kit (Amersham), X-ray film (Fuji Photo Film Co. , Ltd.) was exposed. As a result, it was revealed that NaGLT1 protein localized on renal brush border membrane (Fig. 8). The left part of Fig. 8 shows the result when the reaction was conducted with an anti-NaGLT1 antibody only, and the right part shows the result when the reaction was conducted with an anti-NaGLT1 antibody which had been treated with an antigen peptide.

### (Uptake of fructose by HEK 293 cells mediated by NaGLT1)

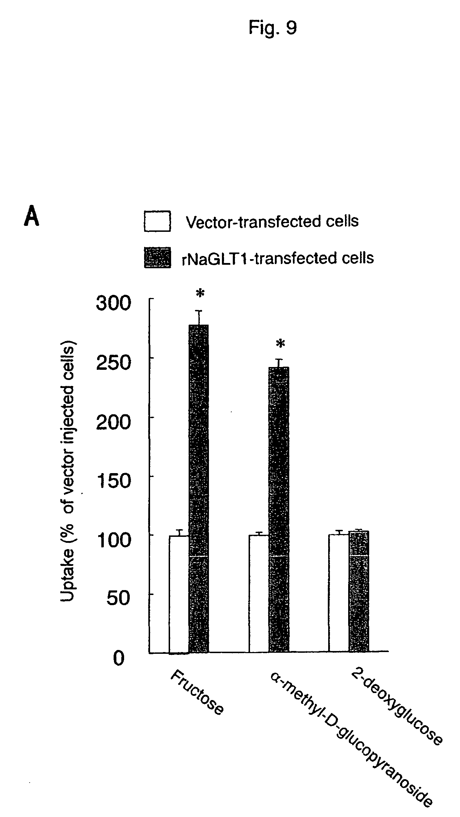

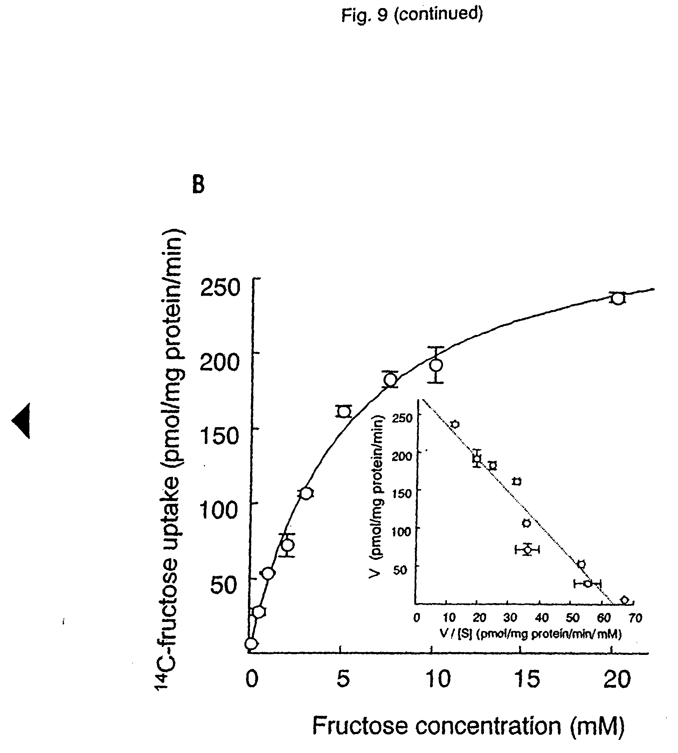

As to HEK293 cells incubated with buffer solution containing glucose analog (0.1 mM, 37 kBq/ml) for 15 minutes at 37°C, uptake analysis was conducted 2 days after the transfection of a vector (white column, 0.8 µg/well) or rNaGLT1 cDNA (black column, 0.8 µg/well) . The results are shown in Fig. 9 (A). Each column indicates mean ± S. E.M obtained from 3 single layers. The value *P < 0.05 was significantly different from that of HEK293 cells transfected with the vector (Student's unpaired t-test).

Uptake of [¹⁴C] fructose by HEK293 cells transfected with rNaGLT1 cDNA (0.8 µg/well) was analyzed in an incubation buffer solution, at various concentrations (0.1 to 20 mM), for 15 minutes at 37°C, and the uptake measured in HEK293 cells transfected with the vector was deducted. The results are shown in Fig. 9 (B) . The inserted graph shows Eadie-Hofstee plot of the uptake . Each point indicates mean ± S.E.M of 3 single layers. The apparent Kₘ and Vₘₐₓ values were obtained from 3 independent experiments.

### (Uptake of fructose by renal brush border membrane vesicles)

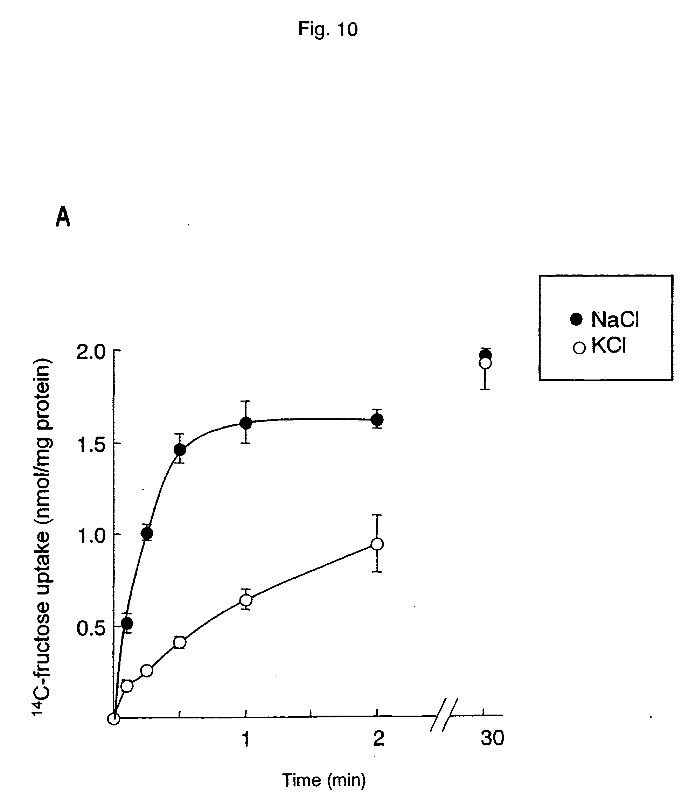

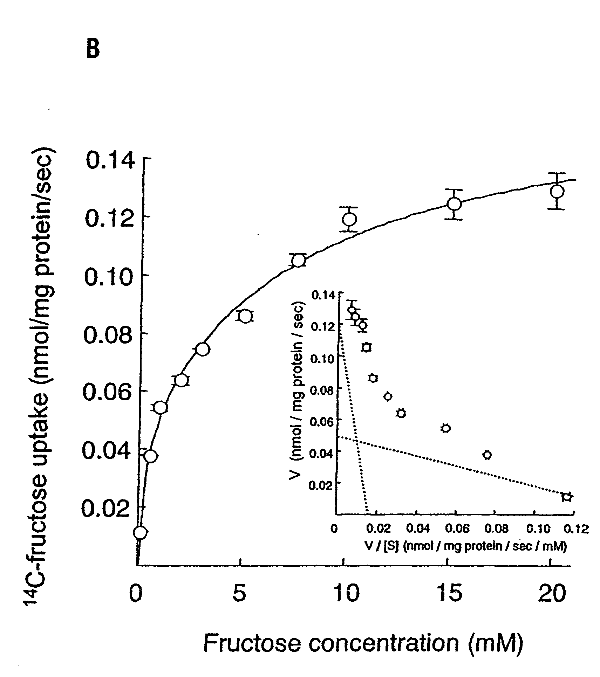

Membrane vesicles (20 µl) suspended in 100 mM mannitol and 10 mM HEPES (pH 7.5) were incubated with a substrate mixture (20 µl) containing 100 mM mannitol, 200 mM NaCl (black circle:•) or KCl (white circle:o), 4 mM [¹⁴C] fructose and 10 mM HEPES (pH 7.5) at 25°C. The results are shown in Fig. 10 (A). The values indicate mean ± S.E.M of 3 independent experiments, respectively. Each experiment was conducted with brush border membrane vesicles isolated from 5 rats.

Na⁺-dependent uptake of fructose by renal brush border membrane vesicles was analyzed in the presence of NaCl in an incubation buffer solution of various concentrations (0.1 to 20 mM) for 15 seconds at 25°C, and the uptake in case where NaCl is substituted with KCl was deduced. The results are shown in Fig. 10 (B). The inserted graph shows Eadie-Hofstee plot of the uptake. Each point indicates mean ± S.E.M of 3 measurements. The apparent Kₘ and Vₘₐₓ values were obtained from 3 independent experiments.

### (Effect of glucose analogs, phloridzin and phloretin, on the uptake of [¹⁴C] fructose by HEK293 cells transfected with rNaGLT1 or renal brush border membrane vesicles)

In the presence and absence of phloridzin (50 µM) or phloretin (50 µM), a glucose analog (30 mM), the uptake of [¹⁴C] fructose (0.1 mM, 37 kBq/ml) by HEK293 cells transfected with rNaGLT1 was measured for 15 minutes at 37°C, and the uptake measured in HEK293 cells transfected with a vector was deducted. In the presence and absence of phloridzin (50 µM) or phloretin (50 µM), a glucose analog (30 mM), membrane vesicles (20 µl) suspended in 100 mM mannitol and 10 mM HEPES (pH7. 5) were incubated with a substrate mixture (20 µl) containing [¹⁴C] fructose (finally, 2 mM, 74 kBq/ml) for 15 seconds at 25°C, and the uptake in case where NaCl is substituted with KCl was deduced. The absence of Na⁺ means the uptake measured under the condition that NaCl is substituted with choline chloride. The results are shown in Table 4. The values indicate mean ± S.E.M of 3 independent experiments, respectively. The value *P < 0.05 was significantly different from that of control (Fisher's t-test).

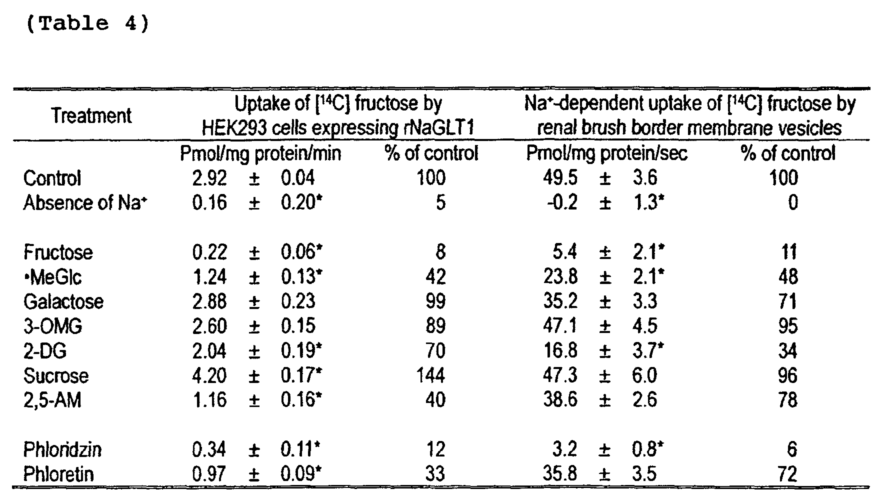

### Industrial Applicability

Renal diabetes is thought to be caused by a defect in renal glucose reabsorption, and the defect in glucose reabsorption is thought to be caused by the deficiency of glucose transporter gene. Because the conventionally unknown gene is obtained by the present invention, it becomes possible to elucidate, diagnose, prevent/treat renal diabetes and to develop drugs for the disease with the use of the gene and a glucose and/or fructose transporter protein, an expression product of the gene.

For example, the isolation of novel genes, diagnosis of glucose and/or fructose transporter function, the detection of genetic diseases in the kidney becomes possible by the isolation of novel glucose and/or fructose transporter genes such as those in the human kidney, and by measuring the expression of glucose and/or fructose transporter in human or rat tissue cells, with the gene and the peptide of the present invention, and further, with an antibody that specifically binds to the peptide.

In addition, it becomes possible to prevent/treat renal genetic diseases by regulating glucose and/or fructose transporter function in tissue cells by introducing the gene or the antisense strand of the gene of the present invention into tissue cells such as renal tissue cells to suppress the expression of the gene . Moreover, a non-human animal model for renal diabetes focused on glucose and/or fructose transporter can be constructed by constructing an animal wherein the gene of the present invention is deficient in its chromosome. By using the non-human animal model, the development of novel drugs for preventing/treating renal diabetes will be possible.

## Claims

1. A DNA which comprises a base sequence shown by SEQ ID NO: 1 in the sequence listing or its complementary sequence, or a sequence containing part or whole of these sequences.

2. A DNA which hybridizes with the DNA according to claim 1 under a stringent condition, and which encodes a polypeptide having glucose and/or fructose transporter function.

3. A DNA which encodes the following polypeptide (a) or (b);
(a) a polypeptide which comprises an amino acid sequence shown by SEQ ID NO: 2 in the sequence listing,
(b) a polypeptide which comprises an amino acid sequence wherein one or a few amino acids are deleted, substituted or added in the amino acid sequence shown by SEQ ID NO: 2 in the sequence listing, and which has glucose and/or fructose transporter function.

4. A polypeptide which comprises an amino acid sequence shown by SEQ ID NO: 2 in the sequence listing.

5. A polypeptide which comprises an amino acid sequence wherein one or a few amino acids are deleted, substituted or added in the amino acid sequence shown by SEQ ID NO: 2 in the sequence listing, and which has glucose and/or fructose transporter function.

6. A method for producing a polypeptide which has glucose and/or fructose transporter function, wherein the DNA according to claims 1 to 3 is incorporated into an expression vector and expressed by introducing the recombinant expression vector into a host cell.

7. An antibodywhich is induced by using the polypeptide according to claim 4 or 5, and which binds to the polypeptide specifically.

8. The antibody according to claim 7, wherein the antibody is a monoclonal antibody.

9. The antibody according to claim 7, wherein the antibody is a polyclonal antibody.

10. A method for producing an animal tissue cell expressing a polypeptide which has glucose and/or fructose transporter function, wherein the DNA according to any one of claims 1 to 3 is introduced into an animal tissue cell.

11. The method for producing an animal tissue cell expressing a polypeptide which has glucose and/or fructose transporter function according to claim 10, wherein the animal tissue cell is a tissue cell of rat kidney, an epithelial cell derived from porcine kidney, an epithelial cell derived from canine kidney or an epithelial cell derived from opossum kidney.

12. The method for producing an animal tissue cell expressing a polypeptide which has glucose and/or fructose transporter function according to claim 10, wherein the animal tissue cell is HEK293, a transfected human embryonic kidney cell line.

13. An animal tissue cell expressing a polypeptide which has glucose and/or fructose transporter function, which is produced by the method according to any one of claims 10 to 12.

14. A method for screening a substance having a glucose and/or fructose transporter function-regulating activity, wherein an effect of a test substance on glucose transport function is measured with the use of the animal tissue cell expressing a polypeptide which has glucose and/or fructose transporter function according to claim 13.

15. A non-human animal model which develops renal diabetes caused by a defect in renal glucose reabsorption, whose gene function to express a polypeptide which has glucose and/or fructose transporter function shown by SEQ ID NO: 2 in the sequence listing is deficient in its chromosome.

16. The non-human animal model which develops renal diabetes according to claim 15 , wherein the deficiency in the gene function to express a polypeptide which has glucose and/or fructose transporter function is deficiency in a function of a gene which expresses a polypeptide which has glucose and/or fructose transporter function shown by SEQ ID NO: 1 in the sequence listing.

17. A method for screening a preventive/therapeutic drug for renal diabetes caused by a defect in glucose reabsorption, wherein a test substance is administered to the non-human animal model which develops renal diabetes caused by a defect in renal glucose and/or fructose reabsorption according to claim 15 or 16, and glucose reabsorption ability of the non-human animal model, or a cell, a tissue or an organ of the non-human animal model is measured/evaluated.

18. A probe for diagnosing glucose and/or fructose transporter function comprising whole or part of an antisense strand of the base sequence according to claim 1.

19. A microarray or a DNA chip for diagnosing glucose and/or fructose transporter function, wherein at least one DNA according to any one of claims 1 to 3 is immobilized.

20. A pharmaceutical for diagnosing glucose and/or fructose transporter function, wherein the antibody according to any one of claims 7 to 9 and/or the probe for diagnosing according to claim 18 is prepared.

21. A method for diagnosing glucose and/or fructose transporter function, wherein a sample is obtained from a test substance, and the expression of the gene according to claim 1 in the sample is measured.

22. A method for diagnosing glucose and/or fructose transporter function, wherein the measurement of the gene expression according to claim 21 is conducted with the probe for diagnosing glucose and/or fructose transporter function according to claim 18 , or with the microarray or the DNA chip for diagnosing glucose and/or fructose transporter function according to claim 19.

23. A method for diagnosing glucose and/or fructose transporter function, wherein a sample is obtained from a test substance and cultured, and the polypeptide according to claim 4 produced by the expression of the gene in the sample is measured.

24. A method for diagnosing glucose and/or fructose transporter function, wherein the measurement of the polypeptide according to claim 23 is conducted with the antibody according to any one of claims 7 to 9.

25. A method for diagnosing a renal disease, wherein the diagnosis of glucose and/or fructose transporter function according to anyone of claims 21 to 24 is measurement of glucose and/or fructose transporter function in a renal disease.

26. A method for regulating glucose and/or fructose transporter function in an animal tissue cell, wherein the DNA according to any one of claims 1 to 3 is introduced into an animal tissue cell.

27. A method for regulating glucose and/or fructose transporter function in an animal tissue cell, wherein the expression of the DNA according to claim 1 is suppressed in an animal tissue cell.

28. A method for regulating glucose and/or fructose transporter function in an animal tissue cell, wherein the expression of the DNA according to claim 1 is suppressed in an animal tissue cell by introducing whole or part of an antisense strand of the DNA base sequence according to claim 1 into an animal tissue cell.

29. The method for regulating glucose and/or fructose transporter function in an animal tissue cell according to any one of claims 26 to 28, wherein the animal tissue cell is an animal kidney cell.


SEQUENCE LISTING

<110> JAPAN SCIENCE AND TECHNOLOGY AGENCY

<120> Glucose and/or fructose transporter 'NaGLT1' and gene thereof

<130> P023477EP

<140> EP 03813300.5
<141> 2003-12-2

<150> PCT/JP03/15418
<151> 2003-12-2

<150> JP P2002-363014
<151> 2002-12-13

<160> 30

<170> PatentIn Ver. 2.1

<210> 1
<211> 2173
<212> DNA
<213> Rattus norvegicus

<220>
<221> CDS
<222> (111)..(1562)

<400> 1
aaagaatctt ctggttagaa agaactgggg ctcagagctc cagggaccct ggcaaaaagc 60

tggacctcac caaaaaccct ttgtctggag ccaccaagct ggggtcggaa atg gag 116
Met Glu
1

ttc cgt ggg tcc ggg gcc act gct gtt gag cag cac ctc ctc cag tcc 164
Phe Arg Gly Ser Gly Ala Thr Ala Val Glu Gln His Leu Leu Gln Ser
        5                   10                  15

gag acc cca ggg aag aat ggg ctg cag gcc aca tcg agt gac caa gtg 212
Glu Thr Pro Gly Lys Asn Gly Leu Gln Ala Thr Ser Ser Asp Gln Val
    20                  25                  30

gga aga aca ctg cgc tgg ttc acc act gtg gtt ctg aat gct gct ttc 260
Gly Arg Thr Leu Arg Trp Phe Thr Thr Val Val Leu Asn Ala Ala Phe
35                  40                  45                  50

ctg gga atg gga gtg agc gct gct gtg ctg gga ccc aca ttt cca gac 308
Leu Gly Met Gly Val Ser Ala Ala Val Leu Gly Pro Thr Phe Pro Asp
                55                  60                  65

ctg gcc aga aac gtg aac cgg aac atc agc agc ctt tcc gaa atc ttc 356
Leu Ala Arg Asn Val Asn Arg Asn Ile Ser Ser Leu Ser Glu Ile Phe
            70                  75                  80

gtg ggc cga gcc ctc ggc tac ctg ggc ggc tct gtg gtt ggt ggg gtg     404
Val Gly Arg Ala Leu Gly Tyr Leu Gly Gly Ser Val Val Gly Gly Val
        85                  90                  95

ctt ttc gac tgc atg aat cat ttt cta ctt ttg ggg ctg tcc cac ctg     452
Leu Phe Asp Cys Met Asn His Phe Leu Leu Leu Gly Leu Ser His Leu
    100                 105                 110

ctt act gcg gcc ggt ctt tac ctc act cct ttc tgt aaa aca gct gcc     500
Leu Thr Ala Ala Gly Leu Tyr Leu Thr Pro Phe Cys Lys Thr Ala Ala
115                 120                 125                 130

tta ctg act gcc atg atg tct att acc ggt gtc tca ttt ggt gtt ctg     548
Leu Leu Thr Ala Met Met Ser Ile Thr Gly Val Ser Phe Gly Val Leu
                135                 140                 145

gat aca ggt ggg aat gtc ctc atc ttg gac ctt tgg ggg gac aaa gga     596
Asp Thr Gly Gly Asn Val Leu Ile Leu Asp Leu Trp Gly Asp Lys Gly
            150                 155                 160

gcc cca cat att cag gcc ttg cac ttc agt ttc gcc ttg ggt gcc ttc     644
Ala Pro His Ile Gln Ala Leu His Phe Ser Phe Ala Leu Gly Ala Phe
        165                 170                 175

ctg gct ccc ctg ctg gct aaa ttg gcc tgg ggt acc aca gca tct gct     692
Leu Ala Pro Leu Leu Ala Lys Leu Ala Trp Gly Thr Thr Ala Ser Ala
    180                 185                 190

cag aac cac aca gag cct cag tta gac cgt tca gcc ttg aac cga tcc     740
Gln Asn His Thr Glu Pro Gln Leu Asp Arg Ser Ala Leu Asn Arg Ser
195                 200                 205                 210

ttt gaa gcc gcc tca gac tct gtg ttg gcg gta cct gac gac atg aat     788
Phe Glu Ala Ala Ser Asp Ser Val Leu Ala Val Pro Asp Asp Met Asn
                215                 220                 225

ctt ctg tgg gcg tac gct tcc att gga acc tat gtt cta gta ctt tct     836
Leu Leu Trp Ala Tyr Ala Ser Ile Gly Thr Tyr Val Leu Val Leu Ser
            230                 235                 240

gtc ttc ctg ttt gct cca ttc ttt aaa aag agg tca aag cag aaa aaa     884
Val Phe Leu Phe Ala Pro Phe Phe Lys Lys Arg Ser Lys Gln Lys Lys
        245                 250                 255

tcc gca gcg tct gct cag gga gct cga agg gct aaa tac cac agg gcc     932
Ser Ala Ala Ser Ala Gln Gly Ala Arg Arg Ala Lys Tyr His Arg Ala
    260                 265                 270

ctg cta tgc ctc ctc ttc ctc ttc ttc ttc ttc tac gtg gga gcg gag     980
Leu Leu Cys Leu Leu Phe Leu Phe Phe Phe Phe Tyr Val Gly Ala Glu
275                 280                 285                 290

gtg acc tac ggc tct tac gta ttc tcc ttc gcc acc acc cac gtt ggc     1028
Val Thr Tyr Gly Ser Tyr Val Phe Ser Phe Ala Thr Thr His Val Gly
                295                 300                 305

atg gaa gag agc gag gca gct ggc ttg aac tcc atc ttc tgg ggg acc     1076

Met Glu Glu Ser Glu Ala Ala Gly Leu Asn Ser Ile Phe Trp Gly Thr
            310                 315                 320

ttc gca gcc tgc agg ggc ctg gcc atc ttc ttc gca acg ctc tta cag 1124
Phe Ala Ala Cys Arg Gly Leu Ala Ile Phe Phe Ala Thr Leu Leu Gln
        325                 330                 335

cct ggg acc atg atg gtg ttg tgt aac att ggc agc ctg gcc tca tct 1172
Pro Gly Thr Met Met Val Leu Cys Asn Ile Gly Ser Leu Ala Ser Ser
    340                 345                 350

ttc ttt ctg gtg ctt ttt gac aag agc cct ctt tgc ctc tgg atc gcg 1220
Phe Phe Leu Val Leu Phe Asp Lys Ser Pro Leu Cys Leu Trp Ile Ala
355                 360                 365                 370

tct tct gtg tat gga gcc tca atg gct gcc acg ttt ccc agc ggc atc 1268
Ser Ser Val Tyr Gly Ala Ser Met Ala Ala Thr Phe Pro Ser Gly Ile
                375                 380                 385

tcc tgg att gag cag tac acc acc tta act ggg aaa tcc gct gcg ttc 1316
Ser Trp Ile Glu Gln Tyr Thr Thr Leu Thr Gly Lys Ser Ala Ala Phe
            390                 395                 400

att ctg gtt ggt gct gcc ctg gga cta atg gcg act cct gca tta tct 1364
Ile Leu Val Gly Ala Ala Leu Gly Leu Met Ala Thr Pro Ala Leu Ser
        405                 410                 415

gga att ctt cag gga cac tat ccc gat ctg cca gta att ctg tac atg 1412
Gly Ile Leu Gln Gly His Tyr Pro Asp Leu Pro Val Ile Leu Tyr Met
    420                 425                 430

tgt ctg ggc tca gca gta tta aca act gtg tta ttc cct gtg atg tat 1460
Cys Leu Gly Ser Ala Val Leu Thr Thr Val Leu Phe Pro Val Met Tyr
435                 440                 445                 450

aaa gta gcc acc tta cct ctg gat cga aag cag gaa aaa agc atc aac 1508
Lys Val Ala Thr Leu Pro Leu Asp Arg Lys Gln Glu Lys Ser Ile Asn
                455                 460                 465

agt gag ggc cag aaa ata tta ctt tct agc tct agg cta atc aag gaa 1556
Ser Glu Gly Gln Lys Ile Leu Leu Ser Ser Ser Arg Leu Ile Lys Glu
            470                 475                 480

gct aaa tgaaagagga aggggaaagg tgtgaaagca cgtgcgcgcg tgtgtgcgca 1612
Ala Lys

tgcacgcgca cgcgtaatgg ttttgcggtg gttaaaatga agaatgggac attctctaat 1672

aaaaatacaa tagaaatgcc tttatataac ccatagctga ggtctctaag caactctcct 1732

gaaatattct gcagccaggg tcttctccag ctgacaggga gcacgcagtc atgaggcacc 1792

aggtctcctg agacccctta cactgccctc attgaagtta tctctcagcc catgattcta 1852

ggaaagaaaa gtatttctaa aataaaatcc acgacttcca gagatcctgt aagacagctc 1912

tgagagatca atgtaactgc cagcaccttc ttcatttcca tgaagtgaga cacagaacag 1972

aaatagtttt aaacgtatgc tcctggggct ggtgagatgg cttagtggtt aagagcactg 2032

actgctcttc caaaggtcct gagttcaaat cccagcaacc acatggtggc tcacaactat 2092

ctgtaatgag atctgatgcc ttcttctggt gtgtctgaag acagcgacag tgtactcata 2152

tacatcaaat aaataatatt t 2173

<210> 2
<211> 484
<212> PRT
<213> Rattus norvegicus

<400> 2
Met Glu Phe Arg Gly Ser Gly Ala Thr Ala Val Glu Gln His Leu Leu
1               5                   10                  15

Gln Ser Glu Thr Pro Gly Lys Asn Gly Leu Gln Ala Thr Ser Ser Asp
            20                  25                  30

Gln Val Gly Arg Thr Leu Arg Trp Phe Thr Thr Val Val Leu Asn Ala
        35                  40                  45

Ala Phe Leu Gly Met Gly Val Ser Ala Ala Val Leu Gly Pro Thr Phe
    50                  55                  60

Pro Asp Leu Ala Arg Asn Val Asn Arg Asn Ile Ser Ser Leu Ser Glu
65                  70                  75                  80

Ile Phe Val Gly Arg Ala Leu Gly Tyr Leu Gly Gly Ser Val Val Gly
                85                  90                  95

Gly Val Leu Phe Asp Cys Met Asn His Phe Leu Leu Leu Gly Leu Ser
            100                 105                 110

His Leu Leu Thr Ala Ala Gly Leu Tyr Leu Thr Pro Phe Cys Lys Thr
        115                 120                 125

Ala Ala Leu Leu Thr Ala Met Met Ser Ile Thr Gly Val Ser Phe Gly
    130                 135                 140

Val Leu Asp Thr Gly Gly Asn Val Leu Ile Leu Asp Leu Trp Gly Asp
145                 150                 155                 160

Lys Gly Ala Pro His Ile Gln Ala Leu His Phe Ser Phe Ala Leu Gly
                165                 170                 175

Ala Phe Leu Ala Pro Leu Leu Ala Lys Leu Ala Trp Gly Thr Thr Ala
            180                 185                 190

Ser Ala Gln Asn His Thr Glu Pro Gln Leu Asp Arg Ser Ala Leu Asn
        195                 200                 205

Arg Ser Phe Glu Ala Ala Ser Asp Ser Val Leu Ala Val Pro Asp Asp
    210                 215                 220

Met Asn Leu Leu Trp Ala Tyr Ala Ser Ile Gly Thr Tyr Val Leu Val
225                 230                 235                 240

Leu Ser Val Phe Leu Phe Ala Pro Phe Phe Lys Lys Arg Ser Lys Gln
                245                 250                 255

Lys Lys Ser Ala Ala Ser Ala Gln Gly Ala Arg Arg Ala Lys Tyr His
            260                 265                 270

Arg Ala Leu Leu Cys Leu Leu Phe Leu Phe Phe Phe Phe Tyr Val Gly
        275                 280                 285

Ala Glu Val Thr Tyr Gly Ser Tyr Val Phe Ser Phe Ala Thr Thr His
    290                 295                 300

Val Gly Met Glu Glu Ser Glu Ala Ala Gly Leu Asn Ser Ile Phe Trp
305                 310                 315                 320

Gly Thr Phe Ala Ala Cys Arg Gly Leu Ala Ile Phe Phe Ala Thr Leu
                325                 330                 335

Leu Gln Pro Gly Thr Met Met Val Leu Cys Asn Ile Gly Ser Leu Ala
            340                 345                 350

Ser Ser Phe Phe Leu Val Leu Phe Asp Lys Ser Pro Leu Cys Leu Trp
        355                 360                 365

Ile Ala Ser Ser Val Tyr Gly Ala Ser Met Ala Ala Thr Phe Pro Ser
    370                 375                 380

Gly Ile Ser Trp Ile Glu Gln Tyr Thr Thr Leu Thr Gly Lys Ser Ala
385                 390                 395                 400

Ala Phe Ile Leu Val Gly Ala Ala Leu Gly Leu Met Ala Thr Pro Ala
                405                 410                 415

Leu Ser Gly Ile Leu Gln Gly His Tyr Pro Asp Leu Pro Val Ile Leu
            420                 425                 430

Tyr Met Cys Leu Gly Ser Ala Val Leu Thr Thr Val Leu Phe Pro Val
        435                 440                 445

Met Tyr Lys Val Ala Thr Leu Pro Leu Asp Arg Lys Gln Glu Lys Ser
    450                 455                 460

Ile Asn Ser Glu Gly Gln Lys Ile Leu Leu Ser Ser Ser Arg Leu Ile
465                 470                 475                 480

Lys Glu Ala Lys

<210> 3
<211> 20
<212> DNA
<213> Artificial Sequence

<220>
<223> Description of Artificial Sequence:T3-1 forward
      primer

<400> 3
tcggaaatgg agttccgtgg                                                    20


<210> 4
<211> 22
<212> DNA
<213> Artificial Sequence

<220>
<223> Description of Artificial Sequence:T3-2 forward
      primer

<400> 4
agctgcctta ctgactgcca tg                                                 22


<210> 5
<211> 21
<212> DNA
<213> Artificial Sequence

<220>
<223> Description of Artificial Sequence:T3-3 forward
      primer

<400> 5
tacgtattct ccttcgccac c                                                  21


<210> 6
<211> 21
<212> DNA
<213> Artificial Sequence

<220>
<223> Description of Artificial Sequence:T3-4 forward
      primer

<400> 6
tgtgtaacat tggcagcctg g                                                  21


<210> 7
<211> 20
<212> DNA
<213> Artificial Sequence

<220>
<223> Description of Artificial Sequence:T3-5 forward
      primer

<400> 7
taacccatag ctgaggtctc                                                    20


<210> 8
<211> 24
<212> DNA
<213> Artificial Sequence

<220>
<223> Description of Artificial Sequence:T7-1 reverse
      primer

<400> 8
cagatagttg tgagccacca tgtg                                               24


<210> 9
<211> 21
<212> DNA
<213> Artificial Sequence

<220>
<223> Description of Artificial Sequence:T7-2 reverse
      primer

<400> 9
gagttgctta gagacctcag c                                                  21


<210> 10
<211> 21
<212> DNA
<213> Artificial Sequence

<220>
<223> Description of Artificial Sequence:T7-3 reverse
      primer

<400> 10
aggtggtgta ctgctcaatc c                                                  21


<210> 11
<211> 22
<212> DNA
<213> Artificial Sequence

<220>
<223> Description of Artificial Sequence:T7-4 reverse
      primer

<400> 11
tctgaggcgg cttcaaagga tc                                                 22


<210> 12

<211> 22
<212> DNA
<213> Artificial Sequence

<220>
<223> Description of Artificial Sequence:T7-5 reverse
      primer

<400> 12
aaaagcaccc caccaaccac ag                                                22


<210> 13
<211> 20
<212> DNA
<213> Artificial Sequence

<220>
<223> Description of Artificial Sequence:NaGLT1 sense
      primer

<400> 13
tgggacccac atttccagac                                                   20


<210> 14
<211> 22
<212> DNA
<213> Artificial Sequence

<220>
<223> Description of Artificial Sequence:NaGLT1
      antisense primer

<400> 14
tctgaggcgg cttcaaagga tc                                                22


<210> 15
<211> 22
<212> DNA
<213> Artificial Sequence

<220>
<223> Description of Artificial Sequence:Rat SGLT1
      sense primer

<400> 15
atggacagta gcaccttgag cc                                                22


<210> 16
<211> 22
<212> DNA
<213> Artificial Sequence

<220>

<223> Description of Artificial Sequence::Rat SGLT1
      antisense primer

<400> 16
tagccccaga gaagatgtct gc                                              22


<210> 17
<211> 22
<212> DNA
<213> Artificial Sequence

<220>
<223> Description of Artificial Sequence:Rat SGLT2 sense
      primer

<400> 17
cattgtctca ggctggcact gg                                              22


<210> 18
<211> 22
<212> DNA
<213> Artificial Sequence

<220>
<223> Description of Artificial Sequence:Rat SGLT2
      antisense primer

<400> 18
ggacactgcc acaatgaaca cc                                              22


<210> 19
<211> 20
<212> DNA
<213> Artificial Sequence

<220>
<223> Description of Artificial Sequence:Rat GAPDH sense
      primer

<400> 19
ccttcattga cctcaactac                                                 20


<210> 20
<211> 20
<212> DNA
<213> Artificial Sequence

<220>
<223> Description of Artificial Sequence:Rat GAPDH
      antisense primer

<400> 20
ggaaggccat gccagtgagc                                                 20

<210> 21
<211> 22
<212> DNA
<213> Artificial Sequence

<220>
<223> Description of Artificial Sequence:NaGLT1 forward
      primer

<400> 21
ccggtgtctc atttggtgtt ct                                                22


<210> 22
<211> 21
<212> DNA
<213> Artificial Sequence

<220>
<223> Description of Artificial Sequence:NaGLT1 reverse
      primer

<400> 22
acccaaggcg aaactgaagt g                                                 21


<210> 23
<211> 28
<212> DNA
<213> Artificial Sequence

<220>
<223> Description of Artificial Sequence:NaGLT1 TaqMan
      probe

<400> 23
acaaaggagc cccacatatt caggcctt                                          28


<210> 24
<211> 23
<212> DNA
<213> Artificial Sequence

<220>
<223> Description of Artificial Sequence:Rat SGLT1
      forward primer

<400> 24
cgaggaggac cctaaagata cca                                               23


<210> 25
<211> 23
<212> DNA

<213> Artificial Sequence

<220>
<223> Description of Artificial Sequence:Rat SGLT1
      reverse primer

<400> 25
gaacaggtca tatgccttcc tga                                              23


<210> 26
<211> 29
<212> DNA
<213> Artificial Sequence

<220>
<223> Description of Artificial Sequence:Rat SGLT1
      TaqMan probe

<400> 26
tgaaatagat gcagaagccc cccagaagg                                        29


<210> 27
<211> 22
<212> DNA
<213> Artificial Sequence

<220>
<223> Description of Artificial Sequence:Rat SGLT2
      forward primer

<400> 27
aaaatacggc aggaaggaac tg                                               22


<210> 28
<211> 21
<212> DNA
<213> Artificial Sequence

<220>
<223> Description of Artificial Sequence:Rat SGLT2
      reverse primer

<400> 28
gacaaattgg ccaccatctt g                                                21


<210> 29
<211> 29
<212> DNA
<213> Artificial Sequence

<220>
<223> Description of Artificial Sequence:Rat SGLT2
      TaqMan probe

<400> 29
ccagtccatt tgattggttg tcacttccc                                        29

<210> 30
<211> 16
<212> PRT
<213> Artificial Sequence

<220>
<223> Description of Artificial Sequence:NaGLT1
      C-terminal peptide

<400> 30
Leu Pro Leu Asp Arg Lys Gln Glu Lys Ser Ile Asn Ser Glu Gly Gln
  1               5                  10                  15

1